# EUROPEAN PATENT SPECIFICATION

(11) **EP 3 358 004 B1**
(45) Date of publication and mention of the grant of the patent: **28.06.2023**
(21) Application number: 16852078.1
(22) Date of filing: 29.09.2016
(51) Int. Cl.: C12N 5/071, G01N 33/50

(54) **METHOD FOR PREPARING SENESCENT MELANOCYTES, CELLS PREPARED BY METHOD, AND METHOD FOR SCREENING FOR SENESCENCE-ALLEVIATING MATERIAL BY USING CELLS**
VERFAHREN ZUR HERSTELLUNG VON SENESZENTEN MELANOZYTEN, NACH DEM VERFAHREN HERGESTELLTE ZELLEN UND VERFAHREN ZUM SCREENING AUF SENESZENZ-LINDERUNGSMATERIAL UNTER VERWENDUNG VON ZELLEN
PROCÉDÉ DE PRÉPARATION DE MÉLANOCYTES SÉNESCENTS, CELLULES PRÉPARÉES PAR LE PROCÉDÉ ET PROCÉDÉ DE CRIBLAGE DE MATÉRIAU ATTÉNUANT LA SÉNESCENCE À L'AIDE DE CELLULES

(30) Priority: 30.09.2015 KR 20150137666
(43) Date of publication of application: 08.08.2018
(73) Proprietor: Amorepacific Corporation, Seoul 04386 (KR)
(72) Inventor: CHOI, Suh-Yeon, Yongin-si Gyeonggi-do 17074 (KR); LEE, Eunkyung, Yongin-si Gyeonggi-do 17074 (KR); CHO, Eun-Gyung, Yongin-si Gyeonggi-do 17074 (KR); LEE, Tae Ryong, Yongin-si Gyeonggi-do17074 (KR)
(74) Representative: Novagraaf Technologies
(86) International application number: PCT/KR2016/010930
(87) International publication number: WO 2017/057932

(56) References cited:
- JP-A- 2010 193 822
- JP-A- 2014 112 073
- JP-A- 2014 520 166
- KR-A- 20070 067 112
- KR-A- 20110 120 030
- ABDEL-MALEK Z ET AL: "Analysis of the UV-Induced Melanogenesis and Growth Arrest of Human Melanocytes", PIGMENT CELL RESEARCH, vol. 7, no. 5, October 1994 (1994-10), pages 326-332, XP55579015, ISSN: 0893-5785
- TOUSSAINT O ET AL: "Perspective Cellular and molecular mechanisms of stress-induced premature senescence (SIPS) of human diploid fibroblasts and melanocytes", EXPERIMENTAL GERONTOLOGY, vol. 35, no. 8, October 2000 (2000-10), pages 927-945, XP55579024, ISSN: 0531-5565, DOI: 10.1016/S0531-5565(00)00180-7
- CHOI S-Y ET AL: "Exposure of human melanocytes to UVB twice and subsequent incubation leads to cellular senescence and senescence-associated pigmentation through the prolonged p53 expression", JOURNAL OF DERMATOLOGICAL SCIENCE, vol. 90, no. 3, June 2018 (2018-06), pages 303-312, XP55579030, AMSTERDAM, NL ISSN: 0923-1811, DOI: 10.1016/j.jdermsci.2018.02.016

## Description

### [Technical Field]

The present specification discloses a method for preparing senescent melanocytes, which can be used in studies on the senescence phenomenon of melanocytes and the senescent pigmentation by repeated exposure to UV rays and culture for a certain period of time and senescent melanocytes prepared by the method.

### [Background Art]

With recent improvements in living standards, modern people are paying more attention to maintenance of healthy skin as well as a healthy body. Hence, there is a growing interest in skin care and alleviation of skin senescence.

Skin is the largest organ of the human body accounting for about 16% of the total human body volume. It is in direct contact with the external environment and functions as an important protective barrier to protect the human body from a number of deadly harmful factors which intrude into the human body, such as temperature, humidity, and UV rays. However, skin cells are damaged by the external environment such as various pollutants and strong UV rays, the cell proliferation is not properly performed, and the skin undergoes wrinkling, loss of elasticity, keratinization, and irregular pigmentation.

Skin senescence is briefly divided into natural senescence (or endogenous senescence) and exogenous senescence, and it is difficult to artificially control natural senescence since natural senescence is affected by genetic factors. However, it is relatively easy to artificially control exogenous senescence since exogenous senescence is affected by environmental factors. As representative exogenous senescence factors,

UV rays, reactive oxygen species, stress, and the like are known. Hence, methods for alleviating exogenous senescence have been recently actively studied, and particularly, efforts for identifying a senescence-preventing or senescence-alleviating material have been continuously made.

JP 2014-520166 A (Bio Spectrum, Inc) 21 August 2014 discloses a test on the effects of madecassoside on melanogenesis of melanocytes, the test characterized by treating, with madecassoside, the melanocytes generated by means of culturing human epidermal melanocytes, derived from an infant, in a medium comprising a human melanocyte growth supplement and then irradiating artificial ultraviolet rays (UVB 20 mJ).

JP 2014-112073 A (Nagoya Univ.) 19 June 2014 discloses a method for screening for an active material with respect to skin senescence by means of senescence-related markers.

Document "Analysis of the UV-Induces Melanogenesis and Growth Arrest of Human Melanocytes", published by Abdel-Malek Z et al., teaches that subjecting cultured melanocytes to two or three rounds of UV irradiation results in a dosedependent decrease in proliferation and morphologic changes which are reminiscent of the alterations observed as melanocytes reach senescence in culture.

### [Summary of Invention]

### [Technical Problem]

In an aspect, an object of the present invention is to provide a method for preparing senescent melanocytes, which can be used for a study on senescent pigmentation.

In another aspect, the present invention provides senescent melanocytes obtained through repeated exposure to UV rays and culture for a certain period of time.

### [Solution to Problem]

In an aspect, the present invention provides a method for preparing senescent melanocytes, the method including irradiating human primary melanocytes, which have been subcultured 1 to 12 times, with UV rays at a strength of 20 to 30 mJ/cm² two or more times; and culturing the melanocytes irradiated with UV rays for 14 days or longer.

### [Advantageous Effects of Invention]

In an aspect, senescent melanocytes prepared according to the method of the present invention can be used for studying a cell senescence phenomenon and a pigmentation mechanism due to the accumulation of UV ray stimulation. In addition, the cells are useful since a material having a senescence-alleviating or skin whitening effect can be screened by using the cells.

### [Brief Description of Drawings]

FIG. 1 is a diagram illustrating the observation results on cell viability depending on the UV strength.
FIG. 2 is a diagram illustrating the cell phenotype depending on the UV strength: senescent cells are indicated by arrows.
FIG. 3a is a diagram illustrating the activity of β-galactosidase depending on the UV strength measured after 2 weeks, FIG. 3b is a diagram illustrating the activity of β-galactosidase stained in 2 weeks after being irradiated with UV rays at 20 mJ/cm² two times, and FIG. 3c is a diagram illustrating the expression of p53 and p21 in 2 weeks after being irradiated with UV rays at 20 mJ/cm² two times (∗; p < 0.05).
FIG. 4 is a diagram illustrating the content of melanin depending on the UV strength (∗; p < 0.05).
FIG. 5a is a diagram illustrating a change in the activity of β-galactosidase after being treated with Whitening Material A and FIG. 5b is a diagram illustrating a change in the content of melanin after being treated with Whitening Material A (∗; p < 0.05).
FIGS. 6a, 6b, and 6c are diagrams illustrating a change in expression of p16 and p21 in the case of being treated with Whitening Materials A, B, and C, respectively and FIG. 6d is a diagram illustrating a change in the melanin content in the case of being treated with Whitening Materials A, B, and C (∗; p < 0.05, ∗∗; p < 0.01).

### [Description of Embodiments]

Hereinafter, the present invention will be described in detail.

In an aspect, the present invention is a method for preparing senescent melanocytes, which includes irradiating human primary melanocytes which have been subcultured 1 to 12 times with UV rays at a strength of 20 to 30 mJ/cm² two or more times ; and culturing the melanocytes irradiated with UV rays for 14 days or longer.

UV rays are generally divided into ultraviolet A (UVA), ultraviolet B (UVB), and ultraviolet C (UVC) by the wavelength. In general, UV rays having wavelengths of 320 to 400 nm are defined as UVA, UV rays having wavelengths of 280 to 320 nm are defined as UVB, and UV rays having wavelengths of 100 nm to 280 nm are defined as UVC.

In the above aspect, the UV rays may be UVB, but the present invention is not limited thereto. For example, the UV rays may be UVA or UVC.

In an embodiment, senescent melanocytes in the most optimized senescent state can be obtained in the case of irradiating the cells with UVB. However, there is no great difference in the effect depending on the wavelength as long as the UV rays to irradiate are included in the region of UVB.

In addition, in the above aspect, the UV strength may be 20 to 30 mJ/cm² or 20 to 25 mJ/cm², and for example, the UV strength may be 20 mJ/cm² or more, 21 mJ/cm² or more, 22 mJ/cm² or more, 23 mJ/cm² or more, or 24 mJ/cm² or more and the UV strength may be 25 mJ/cm² or less, 24 mJ/cm² or less, 23 mJ/cm² or less, 22 mJ/cm² or less, 21 mJ/cm² or less.

In an embodiment, senescent melanocytes in the most optimized senescent state can be obtained when the cell is irradiated with UVB at a strength of 20 mJ/cm².

In the present specification, senescent melanocytes in the most optimized senescent state may refer to, for example, cells exhibiting decreased metabolic activity, such as cells of which senescence may be delayed or may not proceed when being treated with a senescence-alleviating or senescence-preventing material except normal cells which have not undergone senescence and cells which have already undergone cell death and are not alive in a cell clump. In other words, it may refer to melanocytes in a state having an acceptable survival rate without further inducing cell proliferation.

In addition, in the present specification, the senescent pigmentation model may refer to a cell in which pigmentation due to senescence has occurred, and it may be interchangeably used with melanocytes in which the pigmentation is increased together with an increase in the senescence index despite the absence of a stimulating source to induce pigment generation.

In addition, the degree of progress of senescence may be determined by observing, for example, an increase in the expression of p16, p21, p53, and the like known as senescence markers, an increase in the activity of β-galactosidase, a decrease in cell proliferation rate, and a change in the phenotype of cell.

In the present specification, UVB may refer to, for example, an UV ray region having a wavelength of 280 nm to 320 nm.

The cells irradiated with UV rays are cultured for 14 days or longer, 15 days or longer, or 16 days or longer after UV ray irradiation.

In the above aspect, the UV ray irradiation may be conducted two or more times at an interval of 18 hours to 30 hours. The number of UV ray irradiation is not limited and may be two times, three times, or more.

In addition, the interval of UV ray irradiation may be 18 hours to 30 hours. For example, the second UV ray irradiation may be conducted in 17 hours, 18 hours, 19 hours, 20 hours, 21 hours, 22 hours, 23 hours, 23 hours and 30 minutes, 23 hours and 45 minutes, 24 hours, 24 hours and 10 minutes, 24 hours and 15 minutes, 24 hours and 30 minutes, 25 hours, 26 hours, 27 hours, 28 hours, 29 hours, 30 hours, or 31 hours after the first UV ray irradiation.

In addition, in an embodiment, senescent melanocytes in the most optimized senescent state can be obtained in a case in which melanocytes are firstly irradiated with UVB at a strength of 20 mJ/cm² and secondly irradiated under the same conditions as in the first UV ray irradiation in 24 hours after the first UV ray irradiation.

In the above aspects, the cells to be irradiated with UV rays may be human primary melanocytes.

In another aspect, the present invention provides a separated senescent melanocyte prepared by the method described above.

In another aspect, the present invention provides senescent melanocytes for screening a senescence-alleviating or skin whitening material.

In another aspect, the present invention provides senescent melanocytes for screening a senescence-alleviating or skin whitening material.

In the present specification, the senescence index may refer to a gene of which the expression level specifically increases or decreases in a senescent cell and by which the degree of senescence of the cell can be thus confirmed and an expression product of the gene. The senescence index may be, for example, the activity of β-galactosidase or p16, p21 or p53 gene or a protein thereof, and the senescence index includes senescence markers known in the art.

In addition, in the present specification, the whitening index may refer to a gene by which intracellular pigmentation can be confirmed and an expression product of the gene. For example, the whitening index may be melanin.

Hereinafter, the present invention will be described more specifically with reference to the following Examples. However, the following Examples are provided for illustrative purposes only in order to facilitate understanding of the present invention, and the gist and scope of the present invention are not limited thereto.

### [Example 1] Culture of melanocytes and UVB irradiation

Human primary melanocytes (Life Technologies, CA, USA) were cultured in a 5% CO₂ incubator at room temperature by using M-254 medium (Gibco BRL, NY, USA) supplemented with human melanocyte growth supplement (HMGS) (Gibco BRL, NY, USA), placed on a 100 mm culture dish so that the number of cells was 2 × 10⁵, allowed to attach to the dish wall for the night, and irradiated with UVB at a strength of 11 mJ/cm², 20 mJ/cm², and 30 mJ/cm² two times at an interval of 24 hours.

The melanocytes were cultured for up to 2 weeks while changing the medium every 3 days to observe the cytotoxicity, phenotype, senescence index, and the like.

### [Example 2] Observation on cell proliferation and viability of melanocytes after UVB irradiation

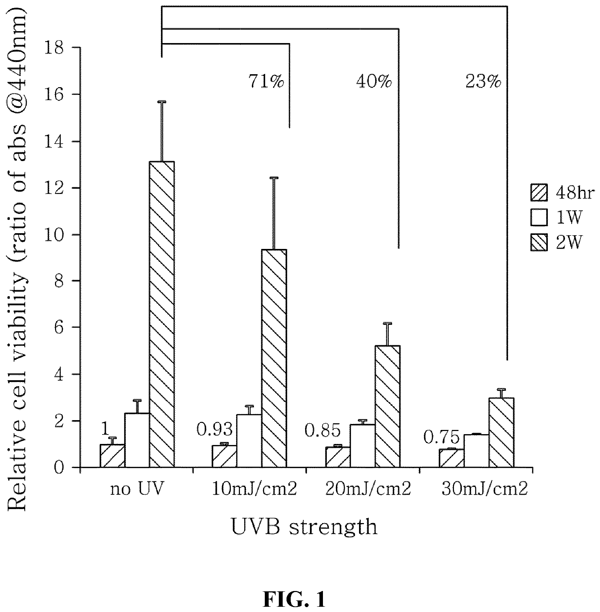

In order to find the UVB irradiation conditions having an acceptable survival rate without further inducing cell proliferation, cell proliferation and viability were investigated by using wst-1 (Roche Applied Science, Germany) solution. The survival rates of cells of Example 1 and cells (control group) cultured without being irradiated with UVB after 48 hours, 1 week, and 2 weeks were observed (FIG. 1).

As a result, it can be seen that the number of cells rapidly increases with time in the case of the control group which has not been treated with UVB. In contrast, the cells irradiated with UVB at a strength of 11 mJ/cm², 20 mJ/cm², and 30 mJ/cm² had survival rates of about 93%, 85%, and 75%, respectively, as compared to the cells of control group in 48 hours after the treatment. In addition, the survival rates were about 71%, 40%, and 23%, respectively, as compared to the control group in 2 weeks after the UVB irradiation. This indicates that the cell proliferation decreases in a UVB strength dependent manner and the proliferation rate is significantly inhibited particularly at a UVB strength of 20 mJ/cm² or more. Decreased proliferation of cells is a representative feature of senescent cells.

### [Example 3] Observation on phenotype of melanocytes in 2 weeks after UVB irradiation

After UVB irradiation under the conditions of Example 1, changes in melanocytes were observed under an optical microscope. The phenotype of cells was observed at a magnification of 40-fold under an optical microscope in an alive state in the medium.

As a result, it has been found that cells having a flattened shape as compared to the initial shape, an increased size of cell body, and an increased number of dendrites, namely, senescent cells have increased among the melanocytes in the case of being irradiated with UVB, the number of these cells have increased in a UVB strength dependent manner, and thus cell heterogeneity has increased as a whole. However, it has been confirmed that there are a great number of cells which have not undergone senescence and have a phenotype similar to that of the group which is not irradiated with UV rays in the group irradiated with UV rays at a strength of 11 mJ/cm² and a relatively small number of alive cells are observed in the group irradiated with UV rays at a strength of 30 mJ/cm².

### [Example 4] Investigation on β-galactosidase activity and p53 and p21 protein expression in melanocytes in 2 weeks after UVB irradiation

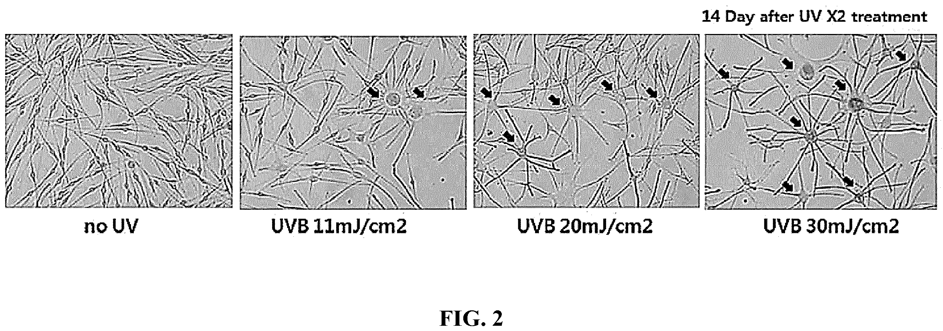

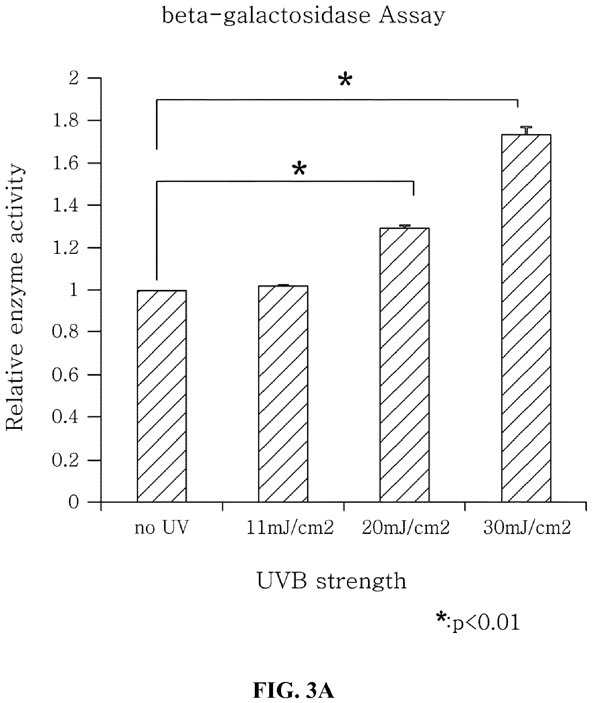

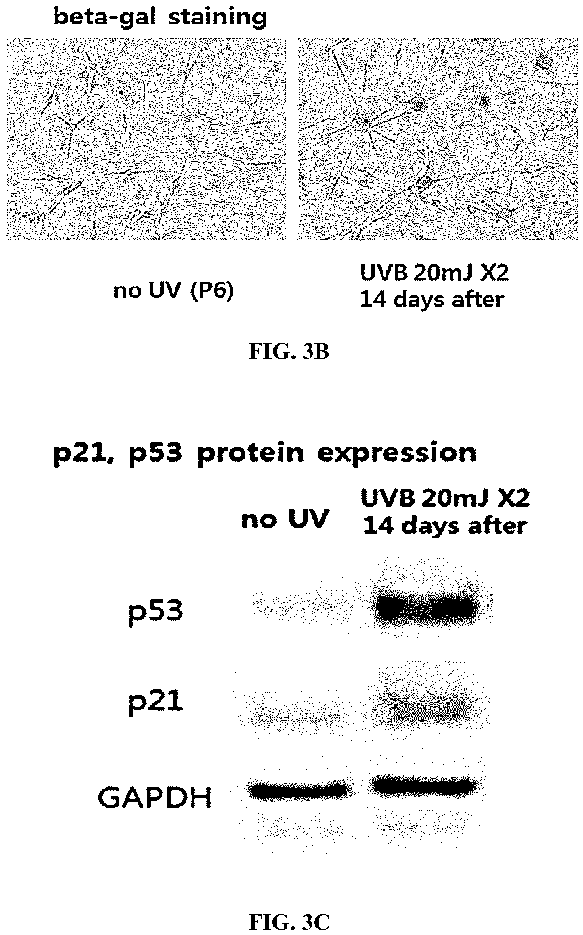

After UVB irradiation under the conditions of Example 1, β-galactosidase activity, which is a representative senescence index of cell, has been measured and it has been confirmed that the activity increased in proportion to the strength of UVB irradiated (FIG. 3a). However, in the case of being irradiated with UVB at a strength of 11 mJ/cm², phenotypic changes due to senescence were clearly observed in some cells, but there are a great number of normal cells, and thus the β-galactosidase activity was not significantly different from that of the control group. In addition, it has been confirmed that cells having the senescent phenotype illustrated in FIG. 2 clearly exhibit β-galactosidase activity as compared to normal cells through a staining experiment (FIG. 3b).

In addition, in order to confirm the expression levels of p53 and p21 proteins, which are known to be expressed in senescent cells in a great amount, cells were irradiated with UVB at the respective strengths and cultured for 2 weeks, detached from the culture dish, then disrupted by adding radioimmunoprecipitation assay buffer (RIPA, Millipore, 20-188) thereto, and subjected to the centrifugation at 13,500 rpm for 30 minutes, the supernatant was separated, and the total proteins were extracted. Western blot analysis was conducted by using a p53-specific antibody (Dako, #M7001) and a p21-specific antibody (cell signaling #2947), and as a result, it has been confirmed that the expression of p53 and p21 proteins has increased after UVB irradiation at a strength of 20 mJ/cm² (FIG. 3c).

### [Example 5] Observation on intracellular melanin accumulation in 2 weeks after UVB irradiation

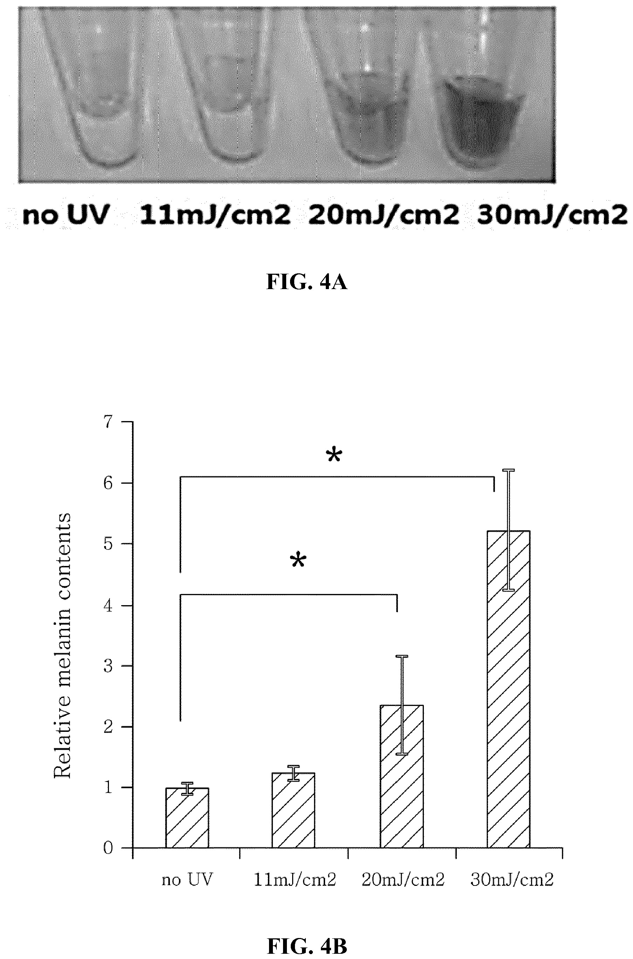

The melanocytes were irradiated with UVB at a strength of 11 mJ/cm², 20 mJ/cm², and 30 mJ/cm², respectively, two times, cultured for 2 weeks, detached from the culture dish and counted, and the same number of cells were collected in a microtube, disrupted by adding RIPA assay buffer (Millipore, 20-188) thereto, and subjected to the centrifugation at 13,500 rpm for 30 minutes to obtain a precipitate. The precipitate was dissolved in a 1N sodium hydroxide (NaOH) solution, and the colors were compared to one another (FIG. 4a), and the absorbance was measured at 450 nm to compare the content of melanin (FIG. 4b).

As a result, it has been confirmed that the content of melanin in melanocytes significantly increases as the strength of UVB increases when melanocytes are irradiated with UVB at a strength of 20 mJ/cm² and 30 mJ/cm². This means that the expression of senescence indexes and the melanin content increase at the same time in a case in which melanocytes are repeatedly irradiated with UVB at a specific strength or higher and then cultured for a specific period of time.

### [Example 6] Observation on mRNA expression level of p16 and p21 in 2 weeks after UVB irradiation

cDNA was prepared by obtaining RNA from cells 2 weeks after being irradiated with UVB at a strength of 20 mJ/cm² two times at an interval of 24 hours, and a change in mRNA level was measured by Q-PCR (Applied biosystems, 7500 Fast) by using p16 and p21 specific primers. As the Q-PCR experiment, a cycle at 95°C for 15 seconds and at 60°C for 60 seconds was repeatedly conducted 40 times in total, and the relative value of an increase in gene expression as compared to that of a control group was measured. The primers used are the following TaqMan products manufactured by Applied Biosystems, respectively: p16 (product number: Hs00923894_m1) and p21 (product number: Hs00355782_m1)

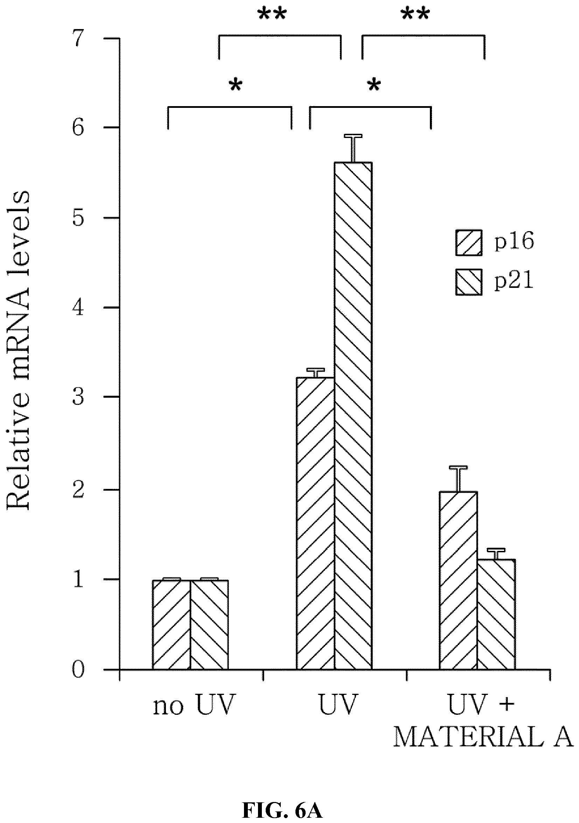

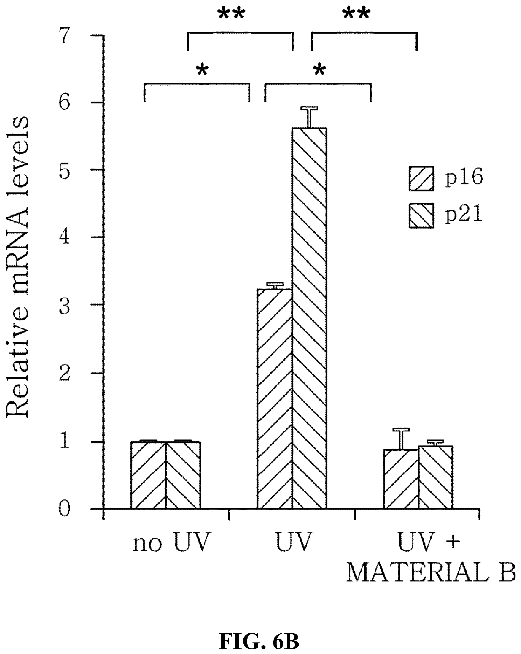

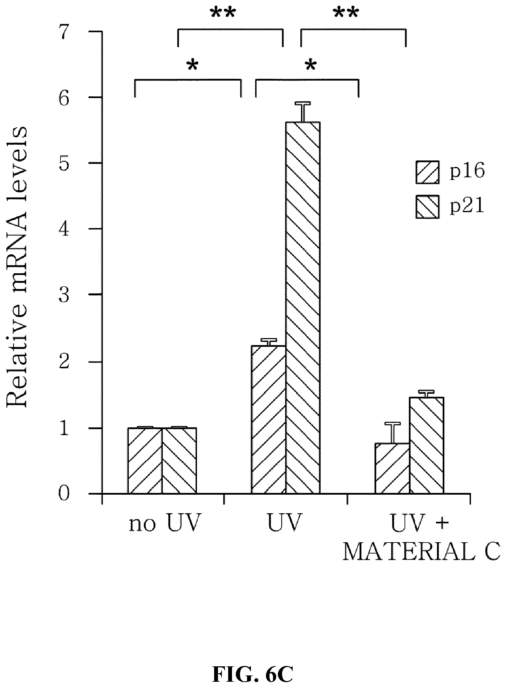

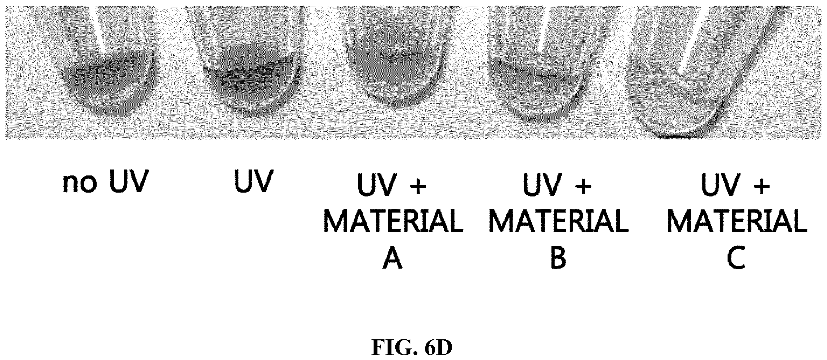

As a result, it has been confirmed that mRNA expression of p16 and p21 greatly increases in a senescent pigmentation model as compared to a control group (FIG. 6).

### [Example 7] Screening of whitening material to alleviate senescent pigmentation: Confirmation of change in melanin content after treatment with whitening material

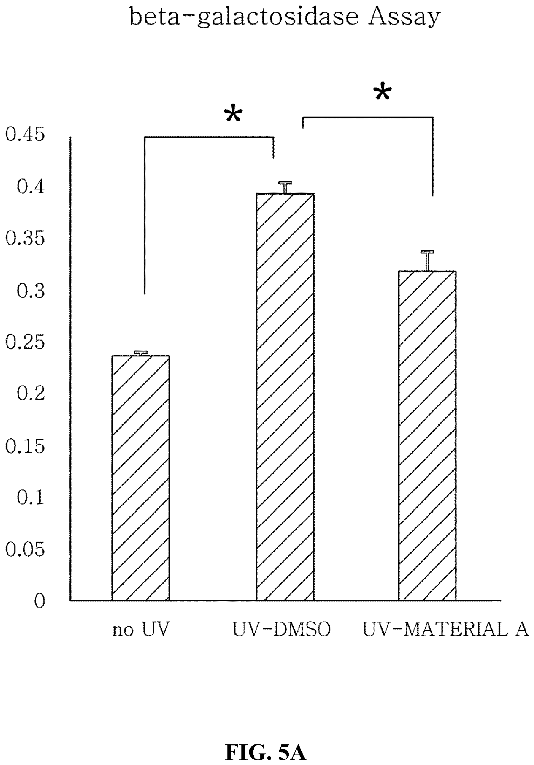

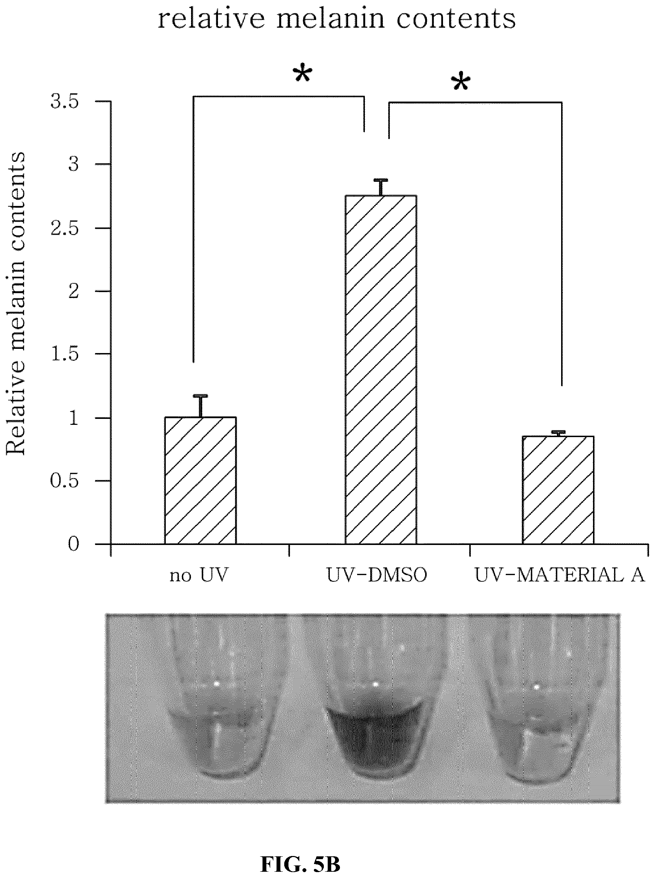

The cells determined in the most proper senescent state through the above-described Examples, namely, the cells prepared by irradiating the melanocytes with UVB at a strength of 20 mJ/cm² two times at an interval of 24 hours were cultured from the 48th hour after the UV ray irradiation in a medium containing Material A (melasolv, 30 µM) of a representative whitening material while changing the medium at an interval of 3 days (4 times in total). After two weeks, the β-galactosidase activity and the melanin content were compared to those of a control group (vehicle (DMSO) treated) (FIG. 5). As a result, it has been confirmed that the β-galactosidase activity increased due to the UVB treatment decreases (FIG. 5a, ∗; p < 0.05) and the melanin content significantly decreases to the level of that of the control group by the treatment with Material A (FIG. 5b, ∗; p < 0.05). From this, it has been confirmed that the senescent cells described above can usefully screen a material which alleviates pigmentation due to senescence of melanocytes.

### [Example 8] Screening whitening material to alleviate senescent pigmentation: Observation on change in expression of p16 and p21 after treatment with whitening material

It has been confirmed that the increased gene expression is significantly inhibited when the cells prepared by irradiating the melanocytes with UVB at a strength of 20 mJ/cm² two times at an interval of 24 hours are treated with Materials A, B, and C, which are previously reported to have a whitening effect, for 2 weeks from 48th hours after the final UVB irradiation four times in total at an interval of 3 days (FIG. 6, *; p < 0.05, ∗∗; p < 0.01). Particularly, the whitening effect has been observed in the case of being treated with Material C which does not exhibit a whitening effect in general pigmentation inducing conditions as well, and this indicates that senescent pigmentation model cells are effective in screening for pigmentation due to senescence of pigment cells.

## Claims

1. A method for preparing senescent melanocytes, the method comprising:
irradiating human primary melanocytes, which have been subcultured 1 to 12 times, with UV rays at a strength of 20 to 30 mJ/cm² two or more times; and
culturing the melanocytes irradiated with UV rays for 14 days or longer.

2. The method for preparing senescent melanocytes according to claim 1, wherein the UV rays are UVB.

3. The method for preparing senescent melanocytes according to claim 1, wherein the UV strength is 20 to 25 mJ/cm².

4. The method for preparing senescent melanocytes according to claim 1, wherein the UV ray irradiation is conducted at an interval of 18 to 30 hours.

5. The method for preparing senescent melanocytes according to claim 1, wherein the UV ray irradiation is conducted two times.

## Patentansprüche

1. Verfahren zur Herstellung von Seneszenzen Melanozyten, wobei das Verfahren umfasst:
Bestrahlung von menschlichen primären Melanozyten, die 1 bis 12 Mal subkultiviert wurden, mit UV-Strahlen mit einer Stärke von 20 bis 30 mJ/cm² zwei oder mehr Mal; und
Kultivierung der mit UV-Strahlen bestrahlten Melanozyten für 14 Tage oder länger.

2. Verfahren zur Herstellung Seneszenzen Melanozyten nach Anspruch 1, wobei die UV-Strahlen UVB sind.

3. Verfahren zur Herstellung Seneszenzen Melanozyten nach Anspruch 1, wobei die UV-Stärke 20 bis 25 mJ/cm² beträgt.

4. Verfahren zur Herstellung Seneszenzen Melanozyten nach Anspruch 1, wobei die UV-Strahlenbestrahlung in einem Intervall von 18 bis 30 Stunden durchgeführt wird.

5. Verfahren zur Herstellung Seneszenzen Melanozyten nach Anspruch 1, wobei die UV-Strahlenbestrahlung zwei Mal durchgeführt wird.

## Revendications

1. Méthode de préparation de mélanocytes sénescents, comprenant :
irradier les mélanocytes primaires humains, qui ont été sous-cultivés de 1 à 12 fois, avec des rayons UV d'une intensité de 20 à 30 mJ/cm² deux fois ou plus ; et
cultiver les mélanocytes irradiés par des rayons UV pendant 14 jours ou plus.

2. Méthode de préparation des mélanocytes sénescents selon la revendication 1, dans laquelle les rayons UV sont des UVB.

3. Méthode de préparation des mélanocytes sénescents selon la revendication 1, dans laquelle l'intensité des UV est de 20 à 25 mJ/cm².

4. Méthode de préparation des mélanocytes sénescents selon la revendication 1, dans laquelle l'irradiation par les rayons UV est effectuée à un intervalle de 18 à 30 heures.

5. Méthode de préparation des mélanocytes sénescents selon la revendication 1, dans laquelle l'irradiation par les rayons UV est effectuée deux fois.
